# EUROPEAN PATENT APPLICATION

(11) **EP 2 857 026 A1**
(43) Date of publication of application: **08.04.2015**
(21) Application number: 13796554.7
(22) Date of filing: 30.05.2013
(51) Int. Cl.: A61K 33/00, A61K 31/05, A61K 31/08, A61K 31/5517, A61K 45/00, A61P 23/00, A61P 25/00, A61P 25/18, A61P 25/28, A61P 43/00

(54) **MEDICINE COMPRISING COMBINATION OF GENERAL ANESTHETIC DRUG AND HYDROGEN**

(30) Priority: 31.05.2012 JP 2012125535
(71) Applicant: Maruishi Pharmaceutical Co., Ltd., Osaka 538-0042 (JP)
(72) Inventor: KAZAMA, Tomiei, Tokorozawa-shi Saitama 359-1132 (JP); SATOH, Yasushi, Tokorozawa-shi Saitama 359-0042 (JP); YONAMINE, Ryuji, Tokorozawa-shi Saitama 359-0042 (JP)
(74) Representative: Witte, Weller & Partner Patentanwälte mbB
(86) International application number: PCT/JP2013/065094
(87) International publication number: WO 2013/180240

(57) **Abstract**

An object of the present invention is to provide a medicine for general anesthesia which can prevent and/or alleviate an anesthetic-induced neurological deficit in the brain (preferably in the developing brain). The present invention relates to a medicine which comprises a combination of a general anesthetic and hydrogen and can prevent and/or alleviate an anesthetic-induced neurological deficit in the brain (preferably in the developing brain).

## Description

### TECHNICAL FIELD

The present invention relates to a medicine comprising a combination of a general anesthetic and hydrogen.

### BACKGROUND ART

There is a concern that neonatal neurological insults cause persistent effects over a long period of time (Non Patent Literature 1, Non Patent Literature 2 and Non Patent Literature 3). For this reason, caution is required for neonatal use of drugs which could potentially alter normal neurodevelopment (for example, substances causing apoptotic neurodegeneration, such as alcohols, phencyclidine, ketamine, N₂O, isoflurane, benzodiazepine, barbiturate and anticonvulsants (Non Patent Literature 4)). Even a single exposure to such drugs is sufficient to induce neurological deficits in neonates, and thus administration of anesthetics also needs attention (Non Patent Literature 5 and Non Patent Literature 6).

Normal neurodevelopment is a carefully regulated sequence of events including proliferation, differentiation, migration and synaptogenesis (Non Patent Literature 7). Glutamate is thought to have a role in all of these processes (Non Patent Literature 8), and for example, high concentrations of glutamate at migration target zones suggest a role as a neuronal chemoattractant (Non Patent Literature 10) along with the NMDA receptor used to detect it (Non Patent Literature 9). The finding of specific NMDA receptor subtypes (e.g. NR2B and NR2D) in different anatomical regions can be helpful for elucidating the precise nature of migration control (Non Patent Literature 10). From work by the same group, it is also apparent that different species employ different mediators in migration control, either GABA (study on rats) or glutamate (study on mice) (Non Patent Literature 11).

Synaptogenesis (brain growth spurt) is a period of a rapid establishment of synapses and is characterized by a high level of programmed cell death (PCD) (up to 1% (Non Patent Literature 12)). This includes the formation of extensive corticothalamic and thalamocortical projections (Non Patent Literature 13). Despite the immense complexity of interspecies embryology, it has been shown that comparisons can be made because the stages in neurodevelopment tend to occur in the same sequence (Non Patent Literature 14). This permits an extrapolation of the period of peak synaptogenic activity from a 7-day-old rat pup (Non Patent Literature 15) to a 0 to 8-month-old human being (Non Patent Literature 16). However, based on analysis of NMDA receptor subtypes, it is more probable that humans experience an extended period of synaptogenesis, i.e. from the beginning of late pregnancy (8 to 10 months of pregnancy) to several years old (Non Patent Literature 17).

Apoptosis, first formally described in 1972 (Non Patent Literature 18), is an essential feature of normal neurodevelopment in processes such as sculpturing, trimming, control of cell numbers and cellular disposal. Apoptosis is characterized as "active cell death" comprising initiation, commitment and execution by dedicated cellular proteins (Non Patent Literature 19).

Programmed cell death (PCD) in the immature central nervous system (CNS) is thought to be controlled by target-derived neurotrophic factors (neurotrophic hypothesis). According to the hypothesis, neurons which have failed to reach their survival promoting synaptic targets (Non Patent Literature 20) initiate, via both neurotrophins and electrical stimulation, a specialized form of cell suicide secondary to withdrawal of environmental trophic support (Non Patent Literature 21 and Non Patent Literature 22). Due to the complex divergent and convergent nature of the "survival pathway," many ligands and mechanisms are involved in maintaining neuronal survival. The cytosol and mitochondria of neurons field a balanced assortment of anti-apoptotic factors (e.g. Bcl-2 and cAMP response element binding protein) and pro-apoptotic factors (e.g. Bad, Bax and the caspase family) which determine cell fate. Bcl-2 and its associated peptides are thought to be particularly important in the developing CNS (Non Patent Literature 23), as evidenced by the high levels of expression in neonates and the fact that experimental over-expression of Bcl-2 can override lack of trophic support (Non Patent Literature 24) and even prevent PCD altogether (Non Patent Literature 25). A variant of Bcl-2 (Bcl-X_{L}) may have a specialized role in maintaining developing neurons before they have found their synaptic targets (Non Patent Literature 26).

In 1999, data were published showing that use of NMDA receptor antagonists in neonatal rats produced specific patterns of neurodegeneration, which were distinct from glial cells (Non Patent Literature 27). On electron microscopy, this neurodegeneration was identical to apoptotic cell death, and most evident in the laterodorsal thalamic nucleus, which is one of the areas of the brain implicated in learning and memory (Non Patent Literature 28). This phenomenon has since been demonstrated in other brain regions with other drugs (Non Patent Literature 29).

Later work showed that neonatal rats are vulnerable to harmful side effects of anesthetics during the synaptogenic period. The neonatal rats demonstrated up to a 68-fold increase in the number of degenerated neurons above the baseline in areas such as the laterodorsal and anteroventral thalamic nuclei, and the parietal cortex after exposure to anesthetics (Non Patent Literature 30). This increase resulted in a functional neurological deficit in behavioral tests later in life. Specifically, the GABAergic anesthetic isoflurane (Non Patent Literature 31) produced dose-dependent neurodegeneration in its own right, and also produced synergistic neurodegeneration by successive addition of midazolam (a double GABAergic cocktail) and then N₂O (a triple cocktail) (Non Patent Literature 30). This process has been shown to occur with exposure to GABAergic agents in areas other than anesthesia, such as anticonvulsant therapy and maternal drug abuse in rats (Non Patent Literature 32 and Non Patent Literature 33).

Since the stages in neurodevelopment occur in the same sequence regardless of the species as described above, despite the interspecies complexity, the effects of anesthetic administration in neonatal rats can be extrapolated to humans to some extent, and human clinical studies have reported many findings on neurotoxicity induced by anesthetic administration in developing brains (Non Patent Literature 34). However, the mechanism of the neurotoxicity induced by anesthetic administration in developing brains involves a number of intricately interrelated factors and is largely unknown. Later work has suggested several neurotoxic mechanisms of anesthetics: (1) increase in apoptosis, (2) effects on GABA neurons, (3) effects on the critical period in cerebral cortex development, etc., and there is also a report that the effects on GABA neurons caused neurological deficits (Non Patent Literature 35). In earlier studies on the neurotoxic mechanism of anesthetics, interest has been focused on apoptosis because of its simple research methodology.

The most important molecule in the intracellular signaling pathway leading to apoptosis is a protease called caspase (Cysteine-ASPartic-acid-proteASE). Activation of caspase-3 initiates apoptosis. Apoptotic signaling pathways are mainly the following ones.
(1) death receptor pathway (tumor necrosis factor receptor (TNFR1) and Fas/CD95 are well known)
(2) mitochondrial pathway (cytochrome c, which is a component of the respiratory electron transport system, plays an important role in the execution of apoptosis as well)
(3) endoplasmic reticulum stress pathway (an apoptotic signal is initiated by events such as production of abnormal proteins in endoplasmic reticulum)
(4) pathway via direct activation of effectors (stressors directly activate effectors without mediation of initiators)

In the death receptor pathway, activation of caspase-8 and caspase-10 occurs. In the mitochondrial pathway, cytochrome c released from mitochondria activates caspase-9. In the endoplasmic reticulum stress pathway, activation of caspase-12 occurs. These initiator caspases activate downstream effector caspases (caspase-3, caspase-6 and caspase-7). In the pathway via direct activation of effectors, direct activation of effector caspases (caspase-3, caspase-6 and caspase-7) occur without mediation of initiator caspases. These caspases cleave poly(ADP ribose) polymerase (PARP) as a substrate, thereby executing apoptosis (Non Patent Literature 36 and Non Patent Literature 37).

The apoptosis possibly induced by anesthetics is thought to have a different mechanism of action from that of ordinary apoptosis, and neither the fundamental mechanism nor effective treatments have been established yet. Therefore, there has been a desire for the development of novel treatments which alleviate anesthetic-induced apoptosis in developing brains and subsequent cognitive dysfunction.

### CITATION LIST

### Non Patent Literature

Non Patent Literature 1: Anand and Scalzo, 2000, Biol. Neonate 77(2): 69-82
Non Patent Literature 2: Balduini et al., 2000, Brain Research 859: 318-325
Non Patent Literature 3: Jevtovic-Todorovic et al., 2003, The Journal of Neuroscience 23(3): 876-882
Non Patent Literature 4: Olney et al., 2002d, Brain Pathol 12(4): 488-498
Non Patent Literature 5: Ikonomidou et al., 2001, Biochemical Pharmacology 62: 401-405
Non Patent Literature 6: Young et al., Cell Death and Differentiation (2003) 10, 1148-1155
Non Patent Literature 7: Butler, 1999, TINS 22(8): 332-334
Non Patent Literature 8: Ikonomidou and Lechoslaw, 2002, Lancet Neurology 1: 383-386
Non Patent Literature 9: Komuro and Rakie, 1993, Science 260(5104): 95-97
Non Patent Literature 10: Behar et al., 1999, The Journal of Neuroscience 19(11): 4449-4461
Non Patent Literature 11: Behar et al., 2001, Cerebral Cortex 11: 744-753
Non Patent Literature 12: Olney et al., 2002b, Neurobiology of Disease 9: 205-219
Non Patent Literature 13: Molar and Blakemore, 1995, Trends Neurosci. 18(9): 389-397
Non Patent Literature 14: Clancy et al., 2001, Neuroscience 105: 7-17
Non Patent Literature 15: Olney et al., 2002a, Neurotoxicology 23(6): 659-668
Non Patent Literature 16: Ikonomidou et al., 1999, Science 238: 70-74
Non Patent Literature 17: Dobbing and Sands, 1979, Early Hum Dev 3: 79-84
Non Patent Literature 18: Kerr et al., 1972, Br J Cancer 26(4): 239-257
Non Patent Literature 19: Sloviter, 2002, TRENDS in Pharmacological Science 23(1): 19-24
Non Patent Literature 20: Sherrard and Bower, 1998, Clin Exp Pharmacol Physiol 25(7-8): 487-495
Non Patent Literature 21: Young et al., 1999, Nature Med 5: 448-453
Non Patent Literature 22: Brenneman et al., 1990, Brain Res Dev Brain Res 51(1): 63-68
Non Patent Literature 23: Yuan and Yanker, 2000, Nature 407: 802-809
Non Patent Literature 24: Garcia et al., 1992, Science 258(5080): 302-304
Non Patent Literature 25: Martinou et al., 1994, Neuron 13(4): 1017-1030
Non Patent Literature 26: Motoyama et al., 1995, Science 267: 1506-1510
Non Patent Literature 27: Ikonomidou et al., 1999, Science 238: 70-74
Non Patent Literature 28: Goen et al., 2002, Behavioural Brain Research 136: 329-337
Non Patent Literature 29: Monti and Contestabile, 2000, European Journal of Neuroscience 12: 3117-3123
Non Patent Literature 30: V. Jevtovic-Todorovic et al., 2003 Journal of Neuroscience 23: 876-882
Non Patent Literature 31: Gyulai et al., 2001, Anesthesiology 95: 585-593
Non Patent Literature 32: Bittigau et al., 2002, PNAS 99(23): 15089-15094
Non Patent Literature 33: Farber and Olney, 2003, Developmental Brain Research 147: 37-45
Non Patent Literature 34: Wilder RT et al., Anesthesiology 100: 796-804, 2009
Non Patent Literature 35: Anesthesiology 2009; 111: 1365-1371
Non Patent Literature 36: Salveen GS, Riedl SJ, 2008 Adv Exp Med Biol. 615: 13-23
Non Patent Literature 37: LA. Pradelli, M. Beneteau, JE. Ricci, 2010 Cell. Mol. Life Sci. 67: 1589-1597

### SUMMARY OF INVENTION

### TECHNICAL PROBLEM

An object of the present invention is to provide a medicine for general anesthesia which can prevent and/or alleviate an anesthetic-induced neurological deficit in the brain (preferably in the developing brain).

### SOLUTION TO PROBLEM

The present inventors conducted extensive research to achieve the above-mentioned object, and as a result, found that a combination of a general anesthetic and hydrogen enables prevention and/or alleviation of an anesthetic-induced neurological deficit in the brain (preferably in the developing brain).

That is, the present invention relates to the following.
[1] A medicine for a human or a non-human animal, comprising a combination of a general anesthetic and hydrogen.
[2] A medicine for general anesthesia of a human or a non-human animal, characterized in that a general anesthetic and hydrogen are administered in combination.
[3] The medicine according to the above [1] or [2], wherein the medicine is used for prevention and/or alleviation of an anesthetic-induced neurological deficit.
[4] The medicine according to the above [3], wherein the anesthetic-induced neurological deficit is associated with neuronal apoptosis.
[5] A medicine for prevention and/or alleviation of an anesthetic-induced neurological deficit, comprising a general anesthetic, the general anesthetic being used in combination with hydrogen.
[6] The medicine according to any one of the above [1] to [5], wherein the general anesthetic is an inhalational anesthetic or a liquid intravenous anesthetic and the hydrogen is hydrogen gas.
[7] The medicine according to the above [6], wherein the concentration of the hydrogen gas in the medicine is 0.15 to 7% (v/v).
[8] The medicine according to any one of the above [1] to [7], wherein the medicine is for a fetus, a neonate, an infant, a preschool child, a child or an elderly adult.
[9] The medicine according to any one of the above [1] to [8], wherein the general anesthetic is one or more kinds of anesthetics selected from the group consisting of nitrous oxide, isoflurane, enflurane, methoxyflurane, sevoflurane, desflurane, diethyl ether, propofol and midazolam.
[10] The medicine according to any one of the above [3] and [5] to [9], wherein the anesthetic-induced neurological deficit is a neuromotor deficit, a neurocognitive deficit, a psychocognitive deficit or autism.
[11] A method for preparing a medicine for prevention and/or alleviation of an anesthetic-induced neurological deficit, the method using a general anesthetic in combination with hydrogen.
[12] The method according to the above [11], wherein the anesthetic-induced neurological deficit is associated with neuronal apoptosis.
[13] The method according to the above [11] or [12], wherein the general anesthetic is an inhalational anesthetic or a liquid intravenous anesthetic and the hydrogen is hydrogen gas.
[14] The method according to the above [13], wherein the concentration of the hydrogen gas in the medicine is 0.15 to 7% (v/v).
[15] The method according to any one of the above [11] to [14], wherein the medicine is for a fetus, a neonate, an infant, a preschool child, a child or an elderly adult.
[16] Use of a general anesthetic for production of a medicine for general anesthesia used in combination with hydrogen.
[17] Use of a general anesthetic and hydrogen for production of a medicine comprising a combination of a general anesthetic and hydrogen.
[18] Use of a general anesthetic and hydrogen for production of a medicine for prevention and/or alleviation of an anesthetic-induced neurological deficit.
[19] The use according to the above [18], wherein the anesthetic-induced neurological deficit is associated with neuronal apoptosis.
[20] The use according to any one of the above [16] to [18], wherein the general anesthetic is an inhalational anesthetic or a liquid intravenous anesthetic and the hydrogen is hydrogen gas.
[21] The use according to the above [20], wherein the concentration of the hydrogen gas in the medicine is 0.15 to 7% (v/v).
[22] The use according to any one of the above [16] to [18], wherein the use is for a fetus, a neonate, an infant, a preschool child, a child or an elderly adult.
[23] The use according to any one of the above [16] to [18], wherein the general anesthetic is one or more kinds of anesthetics selected from the group consisting of nitrous oxide, isoflurane, enflurane, methoxyflurane, sevoflurane, desflurane, diethyl ether, propofol and midazolam.
[24] The use according to the above [18], wherein the anesthetic-induced neurological deficit is a neuromotor deficit, a neurocognitive deficit, a psychocognitive deficit or autism.
[25] A method for preventing and/or alleviating an anesthetic-induced neurological deficit, comprising the step of administering a general anesthetic in combination with hydrogen to a subject.
[26] The method according to the above [25], wherein the general anesthetic is an inhalational anesthetic or a liquid intravenous anesthetic and the hydrogen is hydrogen gas.
[27] The method according to the above [26], wherein the concentration of the hydrogen gas in a medicine is 0.15 to 7% (v/v).
[28] The method according to the above [25], wherein the subj ect is a fetus, a neonate, an infant, a preschool child, a child or an elderly adult.
[29] The method according to the above [25], wherein the general anesthetic is one or more kinds of anesthetics selected from the group consisting of nitrous oxide, isoflurane, enflurane, methoxyflurane, sevoflurane, desflurane, diethyl ether, propofol and midazolam.
[30] The method according to the above [25], wherein the anesthetic-induced neurological deficit is a neuromotor deficit, a neurocognitive deficit, a psychocognitive deficit or autism.
[31] The method according to the above [25], wherein the anesthetic-induced neurological deficit is associated with neuronal apoptosis.

### ADVANTAGEOUS EFFECTS OF INVENTION

The medicine of the present invention enables prevention and/or alleviation of an anesthetic-induced neurological deficit in the brain (preferably in the developing brain). Further, the medicine is convenient, free from side effects, efficacious and inexpensive, and therefore the present invention can provide a medicine for general anesthesia which is effective in medical care in the fields such as obstetrics and pediatrics.

### BRIEF DESCRIPTION OF DRAWINGS

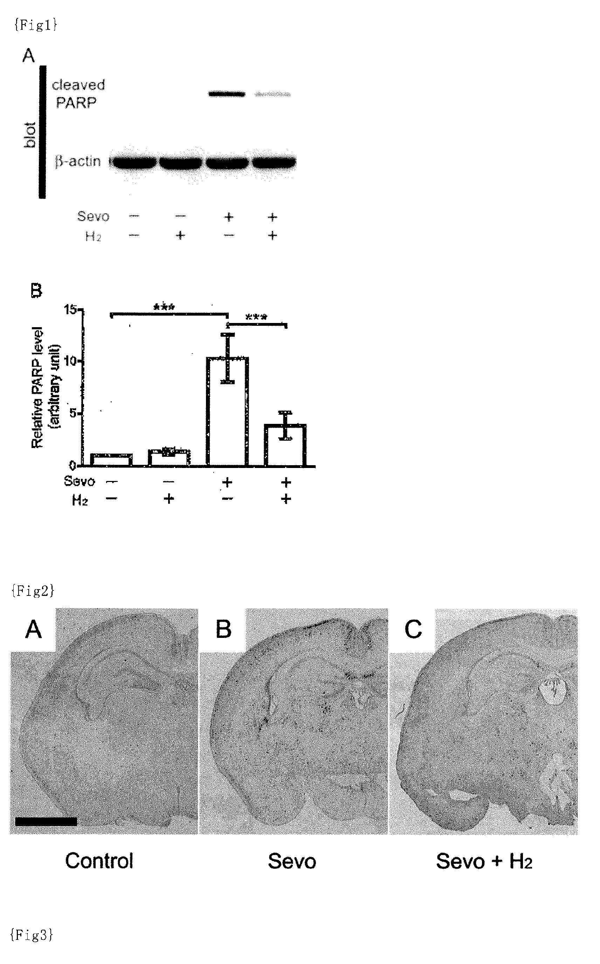

Fig. 1 shows the results of Test Example 1. A shows the results of Western blotting using an antibody against cleaved PARP (biomarker of apoptotic cell death). The β-actin reaction was used as a control. B shows the quantified band intensities of the cleaved PARP. In the figure, *** means P < 0.001. In the figure, Sevo stands for sevoflurane.
Fig. 2 shows optical microscopic images of the mouse brains of Test Example 2. In the figure, A shows the results of the sample of a mouse subjected to administration of 30% oxygen as a carrier gas without sevoflurane (control), B shows an optical microscopic image of the brain of a mouse after 6-hour exposure to 3% sevoflurane with 30% oxygen as a carrier gas, and C shows an optical microscopic image of the brain of a mouse after 6-hour exposure to 3% sevoflurane and 1.3% hydrogen with 30% oxygen as a carrier gas. In the figure, brown spots indicate the presence of cleaved caspase-3-positive cells, i.e., apoptosis. Each image is from one representative mouse out of eight to ten analyzed per group. In the figure, the scale bar marks 1 mm.

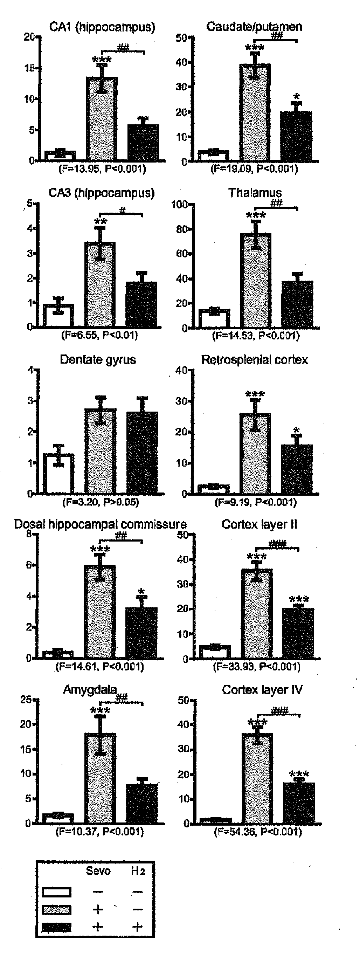

Fig. 3 shows the counts of brown spots representing cleaved caspase-3 detected by immunochemical staining in Test Example 2. Comparison of the mean values of the groups of control, sevoflurane and sevoflurane + hydrogen was performed using a one-way analysis of variance (ANOVA) followed by the Newman-Keuls post-hoc test (n = 8 to 10 mice per group). The F and P values are shown at the bottom of each panel. In the figure, * means P < 0.05, ** means P < 0.01, and *** means P < 0.001 versus the control. # means P < 0.05, ## means P < 0.01, and ### means P < 0.001.

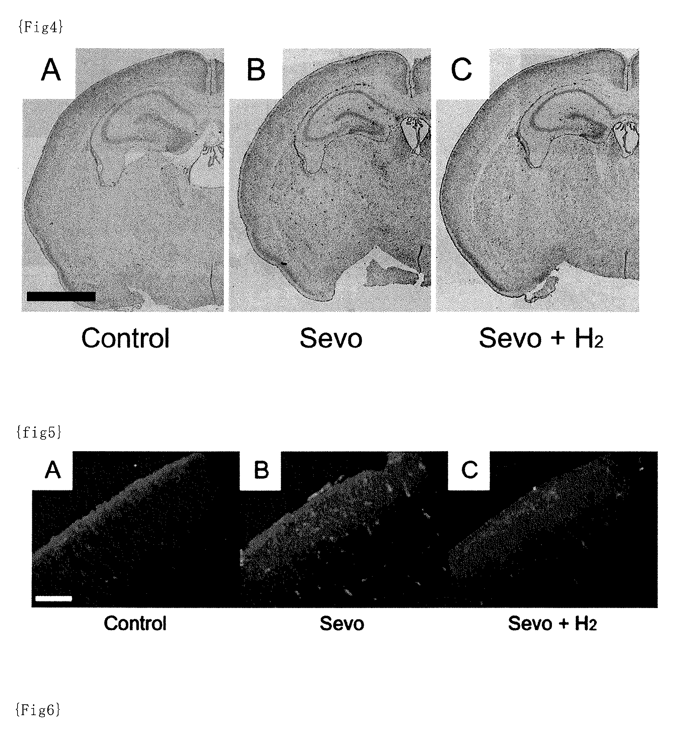

Fig. 4 shows the results of terminal deoxynucleotidyl transferase-mediated nick-end labeling (TUNEL) staining. In the figure, A shows the results of the sample of a mouse subj ected to administration of 30% oxygen as a carrier gas without sevoflurane (control), B shows an optical microscopic image of the brain of a mouse 6 hours after 6-hour exposure to 3% sevoflurane with 30% oxygen as a carrier gas, and C shows an optical microscopic image of the brain of a mouse 6 hours after 6-hour exposure to 3% sevoflurane and 1.3% hydrogen with 30% oxygen as a carrier gas. In the figure, brown spots represent TUNEL-positive cells, i.e. apoptotic cells. Each image is from one representative mouse out of eight analyzed per group. In the figure, the scale bar marks 1 mm.
Fig. 5 shows that hydrogen gas alleviates sevoflurane exposure-induced oxidative stress in the developing brain. In the figure, A shows the results of the sample of a mouse subjected to administration of 30% oxygen as a carrier gas without sevoflurane (control), B shows an optical microscopic image of the brain of a mouse after 6-hour exposure to 3% sevoflurane with 30% oxygen as a carrier gas, and C shows a fluorescence microscopic image of the brain of a mouse after 6-hour exposure to 3% sevoflurane and 1. 3% hydrogen with 30% oxygen as a carrier gas. In the figure, red staining represents 4-hydroxy-2-nonenal (4-HNE) positive cells, i.e. oxidatively stressed cells. In the figure, the scale bar marks 100 µm. Each image is from one representative mouse out of eight analyzed per group.

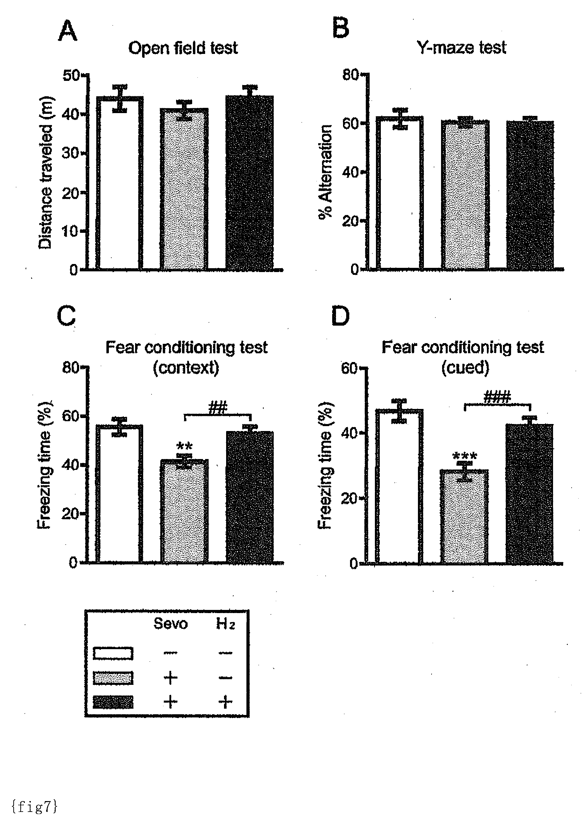

Fig. 6 shows the results of Test Example 3. In the figure, A shows the results of an open field test, B shows the results of a Y-maze test, C shows the results of a contextual fear conditioning test, and D shows the results of an auditory (cued) fear conditioning test. In the figure, ** means P < 0.01 and *** means P < 0.001 versus the control. ## means P < 0.01 and ### means P < 0.001.

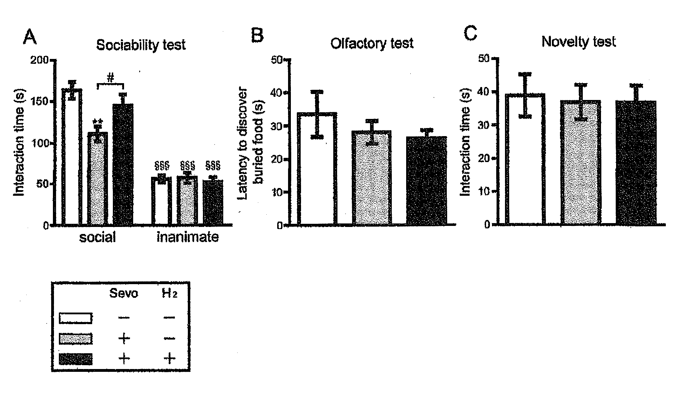

Fig. 7 shows the results of Test Example 3. In the figure, A shows the results of a sociability test, B shows the results of an olfactory test, and C shows the results of a novelty test. In the figure, ** means P < 0.01 and # means P < 0.05 versus the control. §§§ means P < 0.001 versus the corresponding animate target group.

### DESCRIPTION OF EMBODIMENTS

The present invention relates to a medicine for a human or a non-human animal which comprises a combination of a general anesthetic and hydrogen. The present invention also relates to a medicine for general anesthesia of a human or a non-human animal, characterized in that a general anesthetic and hydrogen are administered in combination. The medicine of the present invention can be used for prevention and/or alleviation of an anesthetic-induced neurological deficit. It is suitable that the general anesthetic in the present invention is used in combination with hydrogen. The medicine of the present invention comprises a combination of a general anesthetic and hydrogen, and these components may be separately administered via the same or different administration route at the same time or at a given interval.

As for general anesthetics, it is known in the art that exposure to general anesthetics acting as an NMDA receptor antagonist during the synaptogenic stage of the brain development induces apoptotic neurodegeneration.

Based on the reports that anesthetic exposure increased apoptosis in several regions except for neurons, for example in glial cells (Anesthesiology 2010; 112: 834-841), and that NMDA receptor up-regulation induced apoptosis (Int. J. Devl Neuroscience 27 (2009) 727-731), anesthetics are thought to induce apoptosis via a different mechanism of action from that of ordinary apoptosis, potentially leading to induction of neurological deficits.

Anesthetics having a GABA receptor agonistic action are said to affect GABA neurons and disrupt the balance of excitatory neurons and inhibitory neurons, thereby inducing neurological deficits (Anesthesiology 2009; 111: 1365-1371).

Given the clear implications for pediatric anesthesia and increase in apoptosis level described later, much work is underway to characterize the mechanism behind this process. It is known that activation of both GABA receptors and NMDA receptors affects survival signaling in neuronal cells (Brunet et al., 2001, Current Opinion in Neurobiology 11: 297-305; and Bittigau et al. , 2002, PNAS 99 (23) : 15089-15094), and based on this knowledge, ethanol-intoxicated mice have been used as a basic animal model for study of this process. Caspase-3 is an excellent marker of apoptotic cells, but it is the final effector of the highly divergent death signaling cascade and, due to the position in the cascade, provides little insights into apoptotic mechanisms. Activation of caspase-3 is a common step of both an extrinsic apoptotic pathway mediated by death receptors and an intrinsic apoptotic pathway mediated by mitochondria (Green, 2000, Cell 102: 1-4).

Young et al. attempted narrowing down a search target from the apoptotic mechanisms to a single pathway by a series of proper experiments. A combination of dual immunohistochemistry-immunofluorescence, Western blot analysis and knock-out mice was used to highlight pathway-specific components, particularly Bax and cytochrome c (intrinsic), and caspase-8 (extrinsic) (Young et al., Cell Death and Differentiation (2003) 10, 1148-1155). It was found that ethanol-treated wild type mice showed the characteristic pattern of ethanol-induced apoptosis while homozygous Bax-knockout mice treated in the same manner showed no substantial apoptotic features. Indeed, the level of apoptosis was lower than that seen in the physiological cell death of controls. The absence of caspase-8 activation was also shown in the Bax-knockout mice. Therefore, it was found that the intrinsic apoptotic pathway is involved in anesthetic-induced apoptosis.

The intrinsic pathway centered around mitochondria is controlled by a combination of pro-apoptotic mediators and anti-apoptotic mediators in the cytosols of neuronal cells. In the context of developing neuronal cells, Bcl-X_{L} (a member of the Bcl-2 family) is mainly anti-apoptotic and Bax is pro-apoptotic (Yuan and Yanker, 2000, Nature 407: 802-809). Young et al. made a hypothesis that ethanol, double NMDA receptor antagonists (simultaneous administration of two NMDA receptor antagonists) and a GABAergic anesthetic agent are capable of releasing Bax, which is usually kept in an inactive state in the mitochondrial membrane, to the cytosol.

Once in the cytosol (if unchecked by Bcl-X_{L}), Bax becomes a part of an active complex, which then returns to the mitochondrial membrane and can disrupt the mitochondrial membrane (Korsmeyer et al., 2002, Cell Death and Differentiation 7: 1166-1173). Subsequent translocation of the content in mitochondria (specifically cytochrome c: a part usually responsible for cellular energy production) to the cytosol is considered to produce a very strong pro-apoptotic signal. The cytochrome c in the cytosol forms a complex with Apaf-1 and caspase-8, and the complex then activates caspase-3 to initiate further cascades, finally causing characteristic cleavage of both cytoskeletal proteins and DNAs (Dikranian et al., 2001, Neurobiology of Disease 8: 359-379).

Of course, from this analysis, it is not possible to identify the exact point at which anesthetics interact with this pathway. Also, individual classes of agents are capable of inducing apoptosis (for example, isoflurane alone (Jevtovic-Todorovic et al., 2003) and ketamine alone (Ikonomidou et al., 1999, Science 238: 70-74)), so use of a dual GABAergic agent and NMDA receptor antagonist does not distinguish potential differences between the two receptor interactions, although the ensuing intracellular cascades may converge downstream (Brunet et al., 2001, Current Opinion in Neurobiology 11: 297-305; Bittigau et al. , 2002, PNAS 99 (23) : 15089-15094). It is entirely possible that isoflurane and/or nitrous oxide can dysregulate the intracellular Bax/Bcl-2 ratio, perhaps by disrupting intracellular calcium trafficking.

One possible theory is that the increase in intracellular calcium ion concentration activates a cascade pathway mediated by the activation of calcium ion-dependent enzymes (NOS, PLA2, CaM kinase, etc.) and thereby induces damage of membrane lipids, production of free radical (ROS), failure of ATP production, and mitochondrial respiratory chain dysfunction, which trigger acute or delayed apoptosis. This theory, called the glutamate-calcium ion theory, has been accepted. However, the real causative factor of apoptosis in the cascade of this theory is unclear (Masui "Kyoketsusei shinkei saibou shi no bunshiseibutsugakuteki kijyo to yakubutsu ryouhou niyoru nouhogo" (The Japanese Journal of Anesthesiology, "Molecular Biological Mechanism of Ischemic Neuronal Death and Brain Protection by Medication"), 2007, 56: 248-270).

The general anesthetic in the present invention is not particularly limited as long as it exerts systemic anesthetic effect, and the preferable examples include inhalational anesthetics and intravenous anesthetics.

The inhalational anesthetics in the present invention are not particularly limited, and the examples include volatile inhalational anesthetics such as halothane, isoflurane, enflurane, methoxyflurane, sevoflurane and desflurane; and gaseous inhalational anesthetics such as ethylene, cyclopropane, diethyl ether, chloroform, nitrous oxide and xenon. Preferred are halogenated ether compounds such as isoflurane, enflurane, sevoflurane and desflurane; nitrous oxide; and the like. The inhalational anesthetics may be used in combination with intravenous anesthetics to be administered by injection or intravenous infusion.

The intravenous anesthetics in the present invention are not particularly limited, and the examples include propofol, midazolam, ketamine, tiletamine, thiopental, methohexital and etomidate. Preferred are propofol, midazolam and the like.

More preferably, the general anesthetic used in the present invention is, among the above-listed examples, one or more kinds of anesthetics selected from the group consisting of nitrous oxide, isoflurane, enflurane, methoxyflurane, sevoflurane, desflurane, diethyl ether, propofol and midazolam. Among the above-listed examples of anesthetics, halothane, isoflurane, enflurane, methoxyflurane, sevoflurane, desflurane, etomidate, thiopental, propofol, midazolam, etc. are GABA_{A} receptor agonists. Several of the anesthetics (for example, N₂O, ketamine, isoflurane, etc.) are NMDA receptor antagonists, but the presence of NMDA receptor antagonistic effect has not been confirmed for all anesthetics.

The dose of the general anesthetic varies for every patient depending on the age, the health condition, the interaction with another medicine and the kind of surgical operation to be planned, and is not particularly limited as long as the dose is in such a range that the effects of the present invention can be achieved. For example, the concentration of the general anesthetic such as the above-described inhalational anesthetic and intravenous anesthetic in the medicine may be 0.1 to 10% (v/v), 0.2 to 8% (v/v) or 0.2 to 5% (v/v). The concentration at the beginning of anesthesia may be different from that at the maintenance of anesthetic condition.

In the present invention, hydrogen means a hydrogen molecule (H₂), and any form of a hydrogen molecule may be used without particular limitation. For example, hydrogen gas may be used, and hydrogen water, which is a solution of hydrogen gas in water, may be used.

The subject to whom the general anesthetic and hydrogen are to be applied is not particularly limited, and the examples include animals such as humans, cattle, horses, sheep, goats, dogs, monkeys, cats, bears, rats and rabbits.

The age etc. of the subject to whom the medicine of the present invention is to be applied is not particularly limited, but preferred is a period of life in which an animal subject is susceptible to anesthetics. For example, in the case of a human subject, the subject is preferably a fetus, a neonate, an infant, a preschool child, a child or an elderly adult. Considering the susceptibility of developing brains to anesthetics, more preferred is a fetus, a neonate, an infant, a preschool child, a child or the like, and further preferred is a fetus, a neonate, an infant or a preschool child aged 3 years or younger. The fetus means an unborn baby from 8 weeks after conception until birth. The neonate means a newborn infant under 28 days of age. The infant means a child under 1 year of age. The preschool child means a child aged at least 1 year and less than 7 years. The child means aged at least 7 years and less than 15 years. The elderly adult means a human aged 65 years or older.

In embodiments of the medicine of the present invention, a general anesthetic and hydrogen may be used in combination, and a general anesthetic and hydrogen may be previously mixed.

In the medicine of the present invention, embodiments of the general anesthetic and embodiments of the hydrogen are not particularly limited, but a combination of an inhalational or intravenous anesthetic and hydrogen gas is preferred because such a combination produces remarkable effect on prevention and/or alleviation of an anesthetic-induced neurological deficit.

In the medicine of the present invention, in the case where a general anesthetic and hydrogen are used in combination, the timing for use of the general anesthetic and the timing for use of hydrogen are not particularly limited, and for example, hydrogen may be administered before, simultaneously with, or after general anesthetic administration, and any of these timings may be combined. However, considering that the burden of pretreatment to a subject can be avoided, simultaneous administration of the general anesthetic and hydrogen is preferred. Here, the term "administered before general anesthetic administration" means administering hydrogen for a certain period of time to a subject which has not undergone general anesthetic administration. The term "administered simultaneously with general anesthetic administration" means administering hydrogen to a subject continuously from the beginning to the end of general anesthetic administration, or administering hydrogen to a subject for a given period of time between the beginning and the end of general anesthetic administration. The term "administered after general anesthetic administration" means administering hydrogen to a subject for a given period of time after the end of general anesthetic administration. The durations of general anesthetic administration and of hydrogen administration are not particularly limited, and for example, in the case where sevoflurane at a concentration of 4.0% or lower is used in combination with oxygen and nitrous oxide, the durations may be about 10 minutes to 8 hours.

In the case where a general anesthetic and hydrogen are used in combination, embodiments of the general anesthetic and embodiments of the hydrogen are not particularly limited. In one preferable embodiment of the present invention, the general anesthetic is an inhalational anesthetic or an intravenous anesthetic, and the hydrogen is hydrogen gas because such a combination exerts remarkable effect on prevention and/or alleviation of an anesthetic-induced neurological deficit.

In the medicine of the present invention, in the case where a general anesthetic and hydrogen are previously mixed, the mixing ratio is not particularly limited. For example in the use of an inhalational anesthetic and hydrogen gas, the concentration of the hydrogen gas in the medicine is typically 0.01 to 7% (v/v), and preferably has a reduced upper limit in terms of safety and may be for example 0.15 to 4% (v/v), 0.18 to 3% (v/v), 0.2 to 1.5% (v/v), 0.25% (v/v) or higher and lower than 1% (v/v), or 0.28 to 0.9% (v/v).

The dose of the hydrogen used in the present invention varies for every patient depending on the age, the health condition, the interaction with another medicine and the kind of surgical operation to be planned, and is not particularly limited as long as the dose is in such a range that the effects of the present invention can be achieved. The concentration of the hydrogen in the medicine is typically 0.01 to 7% (v/v), and preferably has a reduced upper limit in terms of safety and may be for example 0.15 to 4% (v/v), 0.18 to 3% (v/v), 0.2 to 1.5% (v/v), 0.25% (v/v) or higher and lower than 1% (v/v), or 0.28 to 0.9% (v/v).

One preferable embodiment of the present invention is a medicine for a human or a non-human animal which comprises a combination of an inhalational anesthetic and hydrogen gas, and the concentration of the hydrogen gas in the medicine, although not subject to any particular limitation, is typically 0.01 to 7% (v/v), and preferably has a reduced upper limit in terms of safety and may be for example 0.15 to 4% (v/v), 0.18 to 3% (v/v), 0.2 to 1.5% (v/v), 0.25% (v/v) or higher and lower than 1% (v/v), or 0.28 to 0.9% (v/v).

One preferable embodiment of the present invention is a medicine for a human or a non-human animal which comprises a combination of a liquid intravenous anesthetic and hydrogen gas, and the concentration of the hydrogen gas in the medicine, although not subject to any particular limitation, is typically 0.01 to 7% (v/v), and preferably has a reduced upper limit in terms of safety and may be for example 0.15 to 4% (v/v), 0.18 to 3% (v/v), 0.2 to 1.5% (v/v), 0.25% (v/v) or higher and lower than 1% (v/v), or 0.28 to 0.9% (v/v).

One preferable embodiment of the present invention is a medicine using an inhalational anesthetic in combination with hydrogen gas, and the concentration of the hydrogen gas in the medicine, although not subject to any particular limitation, is typically 0.01 to 7% (v/v), and preferably has a reduced upper limit in terms of safety and may be for example 0.15 to 4% (v/v), 0.18 to 3% (v/v), 0.2 to 1.5% (v/v), 0.25% (v/v) or higher and lower than 1% (v/v), or 0.28 to 0.9% (v/v).

One preferable embodiment of the present invention is a medicine using a liquid intravenous anesthetic in combination with hydrogen gas, and the concentration of the hydrogen gas in the medicine, although not subject to any particular limitation, is typically 0.01 to 7% (v/v), and preferably has a reduced upper limit in terms of safety and may be for example 0.15 to 4% (v/v), 0.18 to 3% (v/v), 0.2 to 1.5% (v/v), 0.25% (v/v) or higher and lower than 1% (v/v), or 0.28 to 0.9% (v/v).

The medicine of the present invention may comprise oxygen, nitrogen, nitrous oxide or the like unless the effects of the present invention are hindered. The oxygen concentration in the medicine of the present invention is typically about 20 to 90% (v/v), preferably about 20 to 70% (v/v), and more preferably about 20 to 50% (v/v). The concentrations of nitrogen and nitrous oxide are not limited unless the effects of the present invention are hindered.

In the present invention, the gas component(s) in the medicine, except for those described above, may be exclusively nitrogen gas, and may include an atmospheric trace component in addition to nitrogen gas.

Preferable embodiments of the medicine using an inhalational anesthetic and hydrogen gas are not particularly limited and include, for example,
(i) a medicine comprising 0.1 to 10% (v/v) of the inhalational anesthetic, 0.15 to 1.5% (v/v) of hydrogen gas and 20 to 90% (v/v) of oxygen;
(ii) a medicine comprising 0.1 to 8% (v/v) of the inhalational anesthetic, 0.15 to 1.5% (v/v) of hydrogen gas and 20 to 70% (v/v) of oxygen; and
(iii) a medicine comprising 0.1 to 5% (v/v) of the inhalational anesthetic, 0.15 to 1.5% (v/v) of hydrogen gas and 20 to 50% (v/v) of oxygen.

Preferable embodiments of the medicine using a liquid intravenous anesthetic and hydrogen gas are not particularly limited and include, for example,
(i) a medicine comprising 0.1 to 10% (w/w) of the intravenous anesthetic, 0.15 to 1.5% (v/v) of hydrogen gas and 20 to 90% (v/v) of oxygen;
(ii) a medicine comprising 0.1 to 8% (w/w) of the intravenous anesthetic, 0.15 to 1.5% (v/v) of hydrogen gas and 20 to 70% (v/v) of oxygen; and
(iii) a medicine comprising 0.1 to 5% (w/w) of the intravenous anesthetic, 0.15 to 1.5% (v/v) of hydrogen gas and 20 to 50% (v/v) of oxygen.

Another preferable embodiment of the present invention is a medicine for a human or a non-human animal which comprises a combination of an intravenous anesthetic and hydrogen water, and the concentration of the hydrogen water in the medicine is not particularly limited.

Another preferable embodiment of the present invention is a medicine using an intravenous anesthetic in combination with hydrogen water, and the concentration of the hydrogen water in the medicine is not particularly limited.

The medicine of the present invention can prevent and/or alleviate an anesthetic-induced neurological deficit. The term "prevent and/or alleviate a neurological deficit" means reducing the severity of one or more kinds of neurological deficits in a subject (for example, a patient when the subject is a human) to which the medicine of the present invention has been applied, as compared with a subject to which a general anesthetic has been applied in the absence of hydrogen. The term "prevent and/or alleviate a neuronal injury" means reducing the severity of one or more kinds of neuronal injuries in a subject to which the medicine of the present invention has been applied, as compared with a subject to which a general anesthetic has been applied in the absence of hydrogen.

It can be deduced from existing data that the developing human brain undergoes highly dynamic change from a fetal phenotype to a phenotype that resembles the adult one during both the intra-uterine life and the first year of life. This process is characterized by very quick turnover of synapses (as high as 20% per day (Okabe et al., 1999, Nat. Neuroscience 2: 804-811)) and high-level background apoptosis (Hua and Smith, 2004, Nature Neuroscience 7 (4) : 327-332) because neuronal cells which have failed to reach their synaptic target cells are eliminated, presumably based on the preservation of energy efficiency. This study confirms that exposure to anesthetic agents during this crucial stage of neurogenesis (synaptogenesis) induces apoptosis in developing brains. It was experimentally demonstrated that exposure to GABAergic inhalations (for example, isoflurane etc.) induced a 4-fold increase in the apoptosis level in the cortex. Nitrous oxide (nitrous oxide alone causes no neurodegeneration) significantly enhanced isoflurane-induced apoptosis by 12-fold as compared with the control and was confirmed to have neurodegenerative potential. Similar results were observed in the hippocampus, and showed that isoflurane and a mixture of isoflurane and nitrous oxide increased the apoptosis level (4-fold and 7-fold, respectively).

The hippocampus, i.e., a specialized layer of cortical tissue forming part of the limbic system, has an important role in memory formation (Aggleton and Brown, 1999, Behav Brain Sci 22(3): 425-44). Hippocampal neuronal cells have the ability to exhibit the phenomenon known as "long-term potentiation (LTP) ", which is characterized by gradual increase of synaptic efficacy through a specific pattern of neural activity. This process is considered to be the basis of memory at the cellular level. Generally, hippocampal processing takes place in both the hippocampus and the parahippocampal gyrus (subiculum), and the output is relayed to the fornix. Considering that exposure of neonatal rats to a high level of an anesthetic may induce widespread neuronal injuries over the hippocampus and the subiculum, it is not surprising that such rats showed the characteristics of learning deficits in adulthood (Jevtovic-Todorovic et al., 2003), and this finding is supported by detection of LTP suppression in the same study.

The anesthetic-induced neurological deficit in the present invention is preferably an anesthetic-induced neurological deficit in the brain, and examples of the neurological deficit in the present invention include, but are not particularly limited to, a neuromotor deficit, a neurocognitive deficit, a psychocognitive deficit, intellectual disability and autism. The neuromotor deficit includes deficits in strength, balance and mobility. The neurocognitive deficit includes deficits in learning and memory. These neurological deficits may be caused by multiple factors, not a single one, and the possible causative factors include neurodegeneration, neuronal apoptosis and neuronal necrosis. Among them, neuronal apoptosis is considered to affect any of the above deficits. The neurodegeneration means cell shrinkage, chromatin condensation with margination and formation of membrane-enclosed "apoptotic bodies".

The neurocognitive deficit can be usually evaluated according to the following well-established criteria: the short story module of the Randt Memory Test (Randt C, Brown E. Administration manual: Randt Memory Test. New York: Life Sciences, 1983), the digit span subtest and digit symbol subtest of the Wechsler Adult Intelligence Scale-Revised (Wechsler D. The Wechsler Adult Intelligence Scale-Revised (WAIS-R). San Antonio, Tex.: Psychological Corporation, 1981.), the Benton Revised Visual Retention Test (Benton AL, Hansher K. Multilingual aphasia examination. Iowa City: University of Iowa Press, 1978), and the Trail Making Test Part B (Reitan RM. Validity of the Trail Making Test as an indicator of organic brain damage. Percept Mot Skills 1958; 8: 271-6), etc. Other suitable neuromotor and neurocognitive tests are described in Combs D, D'Alecy L: Motor performance in rats exposed to severe forebrain ischemia: Effect of fasting and 1,3-butanediol. Stroke 1987; 18: 503-511; and Gionet T, Thomas J, Warner D, Goodlett C, Wasserman E, West J: Forebrain ischemia induces selective behavioral impairments associated with hippocampal injury in rats. Stroke 1991; 22: 1040-1047.

Another aspect of the present invention relates to a method for preparing a medicine for prevention and/or alleviation of an anesthetic-induced neurological deficit, the method using a general anesthetic in combination with hydrogen. The general anesthetic, the hydrogen, the subject to whom the medicine is to be applied, the anesthetic-induced neurological deficit and a combination thereof are as described above. The preparation method may comprise the step of using a general anesthetic in combination with hydrogen, and may comprise the step of previously mixing a general anesthetic and hydrogen.

Preferable embodiments of the preparation method using an inhalational anesthetic and hydrogen gas are not particularly limited and include, for example,
(i) a method for preparing a medicine, comprising the step of using an inhalational anesthetic in combination with hydrogen gas or previously mixing an inhalational anesthetic and hydrogen gas to give a medicine comprising 0.1 to 10% (v/v) of the inhalational anesthetic, 0.15 to 1.5% (v/v) of hydrogen gas and 20 to 90% (v/v) of oxygen;
(ii) a method for preparing a medicine, comprising the step of using an inhalational anesthetic in combination with hydrogen gas or previously mixing an inhalational anesthetic and hydrogen gas to give a medicine comprising 0.1 to 8% (v/v) of the inhalational anesthetic, 0.15 to 1.5% (v/v) of hydrogen gas and 20 to 70% (v/v) of oxygen; and
(iii) a method for preparing a medicine, comprising the step of using an inhalational anesthetic in combination with hydrogen gas or previously mixing an inhalational anesthetic and hydrogen gas to give a medicine comprising 0.1 to 5% (v/v) of the inhalational anesthetic, 0.15 to 1.5% (v/v) of hydrogen gas and 20 to 50% (v/v) of oxygen.

Preferable embodiments of the preparation method using a liquid intravenous anesthetic and hydrogen gas are not particularly limited and include, for example,
(i) a method for preparing a medicine, comprising the step of using an intravenous anesthetic in combination with hydrogen gas or previously mixing an intravenous anesthetic and hydrogen gas to give a medicine comprising 0.1 to 10% (w/w) of the intravenous anesthetic, 0.15 to 1.5% (v/v) of hydrogen gas and 20 to 90% (v/v) of oxygen;
(ii) a method for preparing a medicine, comprising the step of using an intravenous anesthetic in combination with hydrogen gas or previously mixing an intravenous anesthetic and hydrogen gas to give a medicine comprising 0.1 to 8% (w/w) of the intravenous anesthetic, 0.15 to 1.5% (v/v) of hydrogen gas and 20 to 70% (v/v) of oxygen; and
(iii) a method for preparing a medicine, comprising the step of using an intravenous anesthetic in combination with hydrogen gas or previously mixing an intravenous anesthetic and hydrogen gas to give a medicine comprising 0.1 to 5% (w/w) of the intravenous anesthetic, 0.15 to 1.5% (v/v) of hydrogen gas and 20 to 50% (v/v) of oxygen.

Another aspect of the present invention is the use of a general anesthetic for the production of a medicine for general anesthesia used in combination with hydrogen. The medicine for general anesthesia may comprise a known excipient and additive for the purpose of the stability of medicinal components, hydration of a patient, and the maintenance of electrolyte balance in a patient. The excipient and additive may be any of those conventionally known unless the effects of the present invention are hindered. For example, a propofol-based anesthetic medicine can contain soybean oil, medium chain fatty acid triglyceride, purified yolk lecithin, concentrated glycerin, sodium oleate, and/or the like. The general anesthetic, the hydrogen, and the subject to whom the medicine is to be applied are as described above.

Preferable embodiments of the use of this aspect in which an inhalational anesthetic and hydrogen gas are used are not particularly limited and include, for example,
(i) the use of a general anesthetic for the production of a medicine for general anesthesia which comprises 0.1 to 10% (v/v) of an inhalational anesthetic, 0.15 to 1.5% (v/v) of hydrogen gas and 20 to 90% (v/v) of oxygen and may further comprise an additive if needed;
(ii) the use of a general anesthetic for the production of a medicine for general anesthesia which comprises 0.1 to 8% (v/v) of an inhalational anesthetic, 0.15 to 1.5% (v/v) of hydrogen gas and 20 to 70% (v/v) of oxygen and may further comprise an additive if needed; and
(iii) the use of a general anesthetic for the production of a medicine for general anesthesia which comprises 0.1 to 5% (v/v) of an inhalational anesthetic, 0.15 to 1.5% (v/v) of hydrogen gas and 20 to 50% (v/v) of oxygen and may further comprise an additive if needed.

Preferable embodiments of the use of this aspect in which a liquid intravenous anesthetic and hydrogen gas are used are not particularly limited and include, for example,
(i) the use of a general anesthetic for the production of a medicine for general anesthesia which comprises 0.1 to 10% (w/w) of an intravenous anesthetic, 0.15 to 1.5% (v/v) of hydrogen gas and 20 to 90% (v/v) of oxygen and may further comprise an additive if needed;
(ii) the use of a general anesthetic for the production of a medicine for general anesthesia which comprises 0.1 to 8% (w/w) of an intravenous anesthetic, 0.15 to 1.5% (v/v) of hydrogen gas and 20 to 70% (v/v) of oxygen and may further comprise an additive if needed; and
(iii) the use of a general anesthetic for the production of a medicine for general anesthesia which comprises 0.1 to 5% (w/w) of an intravenous anesthetic, 0.15 to 1.5% (v/v) of hydrogen gas and 20 to 50% (v/v) of oxygen and may further comprise an additive if needed.

Another aspect of the present invention relates to the use of a general anesthetic and hydrogen for the production of a medicine comprising a combination of the general anesthetic and hydrogen. The general anesthetic, the hydrogen, the subject to whom the medicine is to be applied, and a combination thereof are as described above. In embodiments of this use, a general anesthetic and hydrogen may be used in combination, and a general anesthetic and hydrogen may be previously mixed.

Preferable embodiments of the use of this aspect in which an inhalational anesthetic and hydrogen gas are used are not particularly limited and include, for example,
(i) the use of a general anesthetic and hydrogen for the production of a medicine comprising 0.1 to 10% (v/v) of an inhalational anesthetic, 0.15 to 1.5% (v/v) of hydrogen gas and 20 to 90% (v/v) of oxygen;
(ii) the use of a general anesthetic and hydrogen for the production of a medicine comprising 0.1 to 8% (v/v) of an inhalational anesthetic, 0.15 to 1.5% (v/v) of hydrogen gas and 20 to 70% (v/v) of oxygen; and
(iii) the use of a general anesthetic and hydrogen for the production of a medicine comprising 0.1 to 5% (v/v) of an inhalational anesthetic, 0.15 to 1.5% (v/v) of hydrogen gas and 20 to 50% (v/v) of oxygen.

Preferable embodiments of the use of this aspect in which a liquid intravenous anesthetic and hydrogen gas are used are not particularly limited and include, for example,
(i) the use of a general anesthetic and hydrogen for the production of a medicine comprising 0.1 to 10% (w/w) of an intravenous anesthetic, 0.15 to 1.5% (v/v) of hydrogen gas and 20 to 90% (v/v) of oxygen;
(ii) the use of a general anesthetic and hydrogen for the production of a medicine comprising 0.1 to 8% (w/w) of an intravenous anesthetic, 0.15 to 1.5% (v/v) of hydrogen gas and 20 to 70% (v/v) of oxygen; and
(iii) the use of a general anesthetic and hydrogen for the production of a medicine comprising 0.1 to 5% (w/w) of an intravenous anesthetic, 0.15 to 1.5% (v/v) of hydrogen gas and 20 to 50% (v/v) of oxygen.

Another aspect of the present invention relates to the use of a general anesthetic and hydrogen in the production of a medicine for prevention and/or alleviation of an anesthetic-induced neurological deficit. The general anesthetic, the hydrogen, the subject to whom the medicine is to be applied, the anesthetic-induced neurological deficit and their embodiments, and a combination thereof are as described above.

Preferable embodiments of the use of this aspect in which an inhalational anesthetic and hydrogen gas are used are not particularly limited and include, for example,
(i) the use of a general anesthetic and hydrogen for the production of a medicine for prevention and/or alleviation of an anesthetic-induced neurological deficit, the medicine comprising 0.1 to 10% (v/v) of an inhalational anesthetic, 0.15 to 1.5% (v/v) of hydrogen gas and 20 to 90% (v/v) of oxygen;
(ii) the use of a general anesthetic and hydrogen for the production of a medicine for prevention and/or alleviation of an anesthetic-induced neurological deficit, the medicine comprising 0.1 to 8% (v/v) of an inhalational anesthetic, 0.15 to 1.5% (v/v) of hydrogen gas and 20 to 70% (v/v) of oxygen; and
(iii) the use of a general anesthetic and hydrogen for the production of a medicine for prevention and/or alleviation of an anesthetic-induced neurological deficit, the medicine comprising 0.1 to 5% (v/v) of an inhalational anesthetic, 0.15 to 1.5% (v/v) of hydrogen gas and 20 to 50% (v/v) of oxygen.

Preferable embodiments of the use of this aspect in which a liquid intravenous anesthetic and hydrogen gas are used are not particularly limited and include, for example,
(i) the use of a general anesthetic and hydrogen for the production of a medicine for prevention and/or alleviation of an anesthetic-induced neurological deficit, the medicine comprising 0.1 to 10% (w/w) of an intravenous anesthetic, 0.15 to 1.5% (v/v) of hydrogen gas and 20 to 90% (v/v) of oxygen;
(ii) the use of a general anesthetic and hydrogen for the production of a medicine for prevention and/or alleviation of an anesthetic-induced neurological deficit, the medicine comprising 0.1 to 8% (w/w) of an intravenous anesthetic, 0.15 to 1.5% (v/v) of hydrogen gas and 20 to 70% (v/v) of oxygen; and
(iii) the use of a general anesthetic and hydrogen for the production of a medicine for prevention and/or alleviation of an anesthetic-induced neurological deficit, the medicine comprising 0.1 to 5% (w/w) of an intravenous anesthetic, 0.15 to 1.5% (v/v) of hydrogen gas and 20 to 50% (v/v) of oxygen.

Another aspect of the present invention relates to the use of a general anesthetic and hydrogen for the production of a medicine for prevention and/or alleviation of an anesthetic-induced neurological deficit associated with neuronal apoptosis. The general anesthetic, the hydrogen, the subject to whom the medicine is to be applied, the anesthetic-induced neurological deficit and their embodiments, and a combination thereof are as described above.

Preferable embodiments of the use of this aspect in which an inhalational anesthetic and hydrogen gas are used are not particularly limited and include, for example,
(i) the use of a general anesthetic and hydrogen for the production of a medicine for prevention and/or alleviation of an anesthetic-induced neurological deficit associated with neuronal apoptosis, the medicine comprising 0.1 to 10% (v/v) of an inhalational anesthetic, 0.15 to 1.5% (v/v) of hydrogen gas and 20 to 90% (v/v) of oxygen;
(ii) the use of a general anesthetic and hydrogen for the production of a medicine for prevention and/or alleviation of an anesthetic-induced neurological deficit associated with neuronal apoptosis, the medicine comprising 0.1 to 8% (v/v) of an inhalational anesthetic, 0.15 to 1.5% (v/v) of hydrogen gas and 20 to 70% (v/v) of oxygen; and
(iii) the use of a general anesthetic and hydrogen for the production of a medicine for prevention and/or alleviation of an anesthetic-induced neurological deficit associated with neuronal apoptosis, the medicine comprising 0.1 to 5% (v/v) of an inhalational anesthetic, 0.15 to 1.5% (v/v) of hydrogen gas and 20 to 50% (v/v) of oxygen.

Preferable embodiments of the use of this aspect in which a liquid intravenous anesthetic and hydrogen gas are used are not particularly limited and include, for example,
(i) the use of a general anesthetic and hydrogen for the production of a medicine for prevention and/or alleviation of an anesthetic-induced neurological deficit associated with neuronal apoptosis, the medicine comprising 0.1 to 10% (w/w) of an intravenous anesthetic, 0.15 to 1.5% (v/v) of hydrogen gas and 20 to 90% (v/v) of oxygen;
(ii) the use of a general anesthetic and hydrogen for the production of a medicine for prevention and/or alleviation of an anesthetic-induced neurological deficit associated with neuronal apoptosis, the medicine comprising 0.1 to 8% (w/w) of an intravenous anesthetic, 0.15 to 1.5% (v/v) of hydrogen gas and 20 to 70% (v/v) of oxygen; and
(iii) the use of a general anesthetic and hydrogen for the production of a medicine for prevention and/or alleviation of an anesthetic-induced neurological deficit associated with neuronal apoptosis, the medicine comprising 0.1 to 5% (w/w) of an intravenous anesthetic, 0.15 to 1.5% (v/v) of hydrogen gas and 20 to 50% (v/v) of oxygen.

Yet another aspect of the present invention relates to the use of a general anesthetic and hydrogen in the production of a medicine for prevention and/or alleviation of an anesthetic-induced neuronal injury. The general anesthetic, the hydrogen, the subject to whom the medicine is to be applied, the anesthetic-induced neurological deficit and their embodiments, and a combination thereof are as described above.

Another aspect of the present invention relates to a method for preventing and/or alleviating an anesthetic-induced neurological deficit, comprising the step of administering a general anesthetic in combination with hydrogen to a subject. The general anesthetic, the hydrogen, the anesthetic-induced neurological deficit and a combination thereof are as described above. The method may comprise the step of using a general anesthetic in combination with hydrogen, and may comprise the step of previously mixing a general anesthetic and hydrogen.

Preferable embodiments of the method of this aspect using an inhalational anesthetic and hydrogen gas are not particularly limited and include, for example,
(i) a method for preventing and/or alleviating an anesthetic-induced neurological deficit, comprising the step of administering 0.1 to 10% (v/v) of an inhalational anesthetic, 0.15 to 1.5% (v/v) of hydrogen gas and 20 to 90% (v/v) of oxygen to a subject;
(ii) a method for preventing and/or alleviating an anesthetic-induced neurological deficit, comprising the step of administering 0.1 to 8% (v/v) of an inhalational anesthetic, 0.15 to 1.5% (v/v) of hydrogen gas and 20 to 70% (v/v) of oxygen to a subject; and
(iii) a method for preventing and/or alleviating an anesthetic-induced neurological deficit, comprising the step of administering 0.1 to 5% (v/v) of an inhalational anesthetic, 0.15 to 1.5% (v/v) of hydrogen gas and 20 to 50% (v/v) of oxygen to a subject.

Preferable embodiments of the method of this aspect using a liquid intravenous anesthetic and hydrogen gas are not particularly limited and include, for example,
(i) a method for preventing and/or alleviating an anesthetic-induced neurological deficit, comprising the step of administering 0.1 to 10% (w/w) of an intravenous anesthetic, 0.15 to 1.5% (v/v) of hydrogen gas and 20 to 90% (v/v) of oxygen to a subject;
(ii) a method for preventing and/or alleviating an anesthetic-induced neurological deficit, comprising the step of administering 0.1 to 8% (w/w) of an intravenous anesthetic, 0.15 to 1.5% (v/v) of hydrogen gas and 20 to 70% (v/v) of oxygen to a subject; and
(iii) a method for preventing and/or alleviating an anesthetic-induced neurological deficit, comprising the step of administering 0.1 to 5% (w/w) of an intravenous anesthetic, 0.15 to 1.5% (v/v) of hydrogen gas and 20 to 50% (v/v) of oxygen to a subject.

In the above-described aspects, in the case where a general anesthetic and hydrogen are used in combination, the timing for use of the general anesthetic and the timing for use of hydrogen are not particularly limited, and for example, hydrogen may be administered before, simultaneously with, or after general anesthetic administration, and any of these timings may be combined. However, considering that the burden of pretreatment to a subject can be avoided, simultaneous administration of the general anesthetic and hydrogen is preferred. Here, the term "administered before general anesthetic administration" means administering hydrogen for a certain period of time to a subject which has not undergone general anesthetic administration. The term "administered simultaneously with general anesthetic administration" means administering hydrogen to a subject continuously from the beginning to the end of general anesthetic administration, or administering hydrogen to a subject for a given period of time between the beginning and the end of general anesthetic administration. The term "administered after general anesthetic administration" means administering hydrogen to a subject for a given period of time after the end of general anesthetic administration. The durations of general anesthetic administration and of hydrogen administration are not particularly limited. The subject to whom the general anesthetic and hydrogen are to be administered is not particularly limited, and the examples include animals such as humans, cattle, horses, sheep, goats, dogs, monkeys, cats, bears, rats and rabbits.

The age etc. of the subject to whom the general anesthetic and hydrogen are to be administered is not particularly limited, but preferred is a period of life in which an animal subject is susceptible to anesthetics. For example, in the case of a human subject, the subject is preferably a fetus, a neonate, an infant, a preschool child, a child or an elderly adult. Considering the susceptibility of developing brains to anesthetics, more preferred is a fetus, a neonate, an infant, a preschool child, a child or the like, and further preferred is a fetus, a neonate, an infant or a preschool child aged 3 years or younger. The definitions of the fetus, the neonate, the infant, the preschool child, the child and the elderly adult are as described above.

Another aspect of the present invention relates to a method for inhibiting anesthetic-induced apoptosis, comprising the step of administering a medicine comprising a combination of a general anesthetic and hydrogen to a subject. The general anesthetic, the hydrogen, the subject to whom the medicine is to be applied, and a combination thereof are as described above. The method may comprise the step of using a general anesthetic in combination with hydrogen, and may comprise the step of previously mixing a general anesthetic and hydrogen.

Preferable embodiments of the inhibition method using an inhalational anesthetic and hydrogen gas are not particularly limited and include, for example,
(i) a method for inhibiting anesthetic-induced apoptosis comprising the steps of using an inhalational anesthetic in combination with hydrogen gas or previously mixing an inhalational anesthetic and hydrogen gas to give a medicine comprising 0.1 to 10% (v/v) of the inhalational anesthetic, 0.15 to 1.5% (v/v) of hydrogen gas and 20 to 90% (v/v) of oxygen, and administering the medicine obtained in the above step to a subject;
(ii) a method for inhibiting anesthetic-induced apoptosis comprising the steps of using an inhalational anesthetic in combination with hydrogen gas or previously mixing an inhalational anesthetic and hydrogen gas to give a medicine comprising 0.1 to 8% (v/v) of the inhalational anesthetic, 0.15 to 1.5% (v/v) of hydrogen gas and 20 to 70% (v/v) of oxygen, and administering the medicine obtained in the above step to a subject; and
(iii) a method for inhibiting anesthetic-induced apoptosis comprising the steps of using an inhalational anesthetic in combination with hydrogen gas or previously mixing an inhalational anesthetic and hydrogen gas to give a medicine comprising 0.1 to 5% (v/v) of the inhalational anesthetic, 0.15 to 1.5% (v/v) of hydrogen gas and 20 to 50% (v/v) of oxygen, and administering the medicine obtained in the above step to a subject.

Preferable embodiments of the inhibition method using a liquid intravenous anesthetic and hydrogen gas are not particularly limited and include, for example,
(i) a method for inhibiting anesthetic-induced apoptosis comprising the steps of using an intravenous anesthetic in combination with hydrogen gas or previously mixing an intravenous anesthetic and hydrogen gas to give a medicine comprising 0.1 to 10% (w/w) of the intravenous anesthetic, 0.15 to 1.5% (v/v) of hydrogen gas and 20 to 90% (v/v) of oxygen, and administering the medicine obtained in the above step to a subject;
(ii) a method for inhibiting anesthetic-induced apoptosis comprising the steps of using an intravenous anesthetic in combination with hydrogen gas or previously mixing an intravenous anesthetic and hydrogen gas to give a medicine comprising 0.1 to 8% (w/w) of the intravenous anesthetic, 0.15 to 1.5% (v/v) of hydrogen gas and 20 to 70% (v/v) of oxygen, and administering the medicine obtained in the above step to a subject; and
(iii) a method for inhibiting anesthetic-induced apoptosis comprising the steps of using an intravenous anesthetic in combination with hydrogen gas or previously mixing an intravenous anesthetic and hydrogen gas to give a medicine comprising 0.1 to 5% (w/w) of the intravenous anesthetic, 0.15 to 1.5% (v/v) of hydrogen gas and 20 to 50% (v/v) of oxygen, and administering the medicine obtained in the above step to a subject.

In the present invention, the anesthetic-induced neurological deficit was evaluated by apoptosis assays and behavioral tests. The apoptosis assays were (i) cleaved PARP quantification, (ii) active caspase-3 staining, and (iii) TUNEL assay.

In the present invention, western blot analysis was used for detection and quantification of cleaved PARP. One of the key initiation factors of the apoptotic cascade is the activation of caspases and the subsequent cleavage of poly (adenosine diphosphate ribose) polymerase (PARP). PARP is an intranuclear enzyme which is normally involved in DNA repair, DNA stability and other intracellular events, and the final target of caspase-3 in the apoptotic cascade. In contrast to measuring the active caspase, which is degraded during apoptosis, measuring cleaved PARP allows sustained signal detection even in late stages of apoptosis.

In the present invention, staining of active caspase-3 was performed by immunohistochemical analysis for caspase-3. At the end of the apoptotic signaling cascade, caspase-9 activates caspase-3 (cysteine protease). Thus, caspase-3 is a marker of cells that are downstream of the apoptotic commitment point. The immunohistochemical analysis commonly performed in parallel with silver staining serves as a marker suitable for neuronal apoptosis and is excellent for both quantification and characterization of physiological cell death (Olney et al., 2002b, Neurobiology of Disease 9: 205-219). Caspase-3 is a cytoplasmic enzyme, and thus active caspase-3-stained cells are stained in their entirety, hence making quantification relatively easy.

In the present invention, DNA fragmentation in early stages of apoptosis was visualized by TUNEL assay. The DNA fragmentation includes double-strand breaks and single-strand breaks. Both types of breaks can be detected by labeling the free 3'-OH termini of the fragments with modified nucleotides in an enzymatic reaction. The TUNEL assay is used as a highly sensitive detection method for apoptosis.

### EXAMPLES

Next, the present invention will be illustrated in more detail by examples, but is not limited thereto. Within the scope of the technical idea of the present invention, various modifications can be made by persons of ordinary knowledge in the art.

Statistical analysis in the following examples was performed using GraphPad Prism 5 (GraphPad Software Inc., La Jolla, CA). Comparison of the mean values of the groups was performed by a one-way analysis of variance (ANOVA) followed by the Newman-Keuls post-hoc test or a two-way analysis of variance (ANOVA) followed by the Bonferroni post-hoc test. In the Y-maze test, comparison of group performance relative to random performance was performed using a two-tailed one-sample t-test. When the P value was < 0.05, the difference was regarded as statistically significant. The values are given as the mean and the standard error of the mean.

All experiments were conducted according to the ethical guidelines for animal experiments of the National Defense Medical College and approved by the Committee for Animal Research at the National Defense Medical College (Tokorozawa, Saitama, Japan).

### <Example 1>

Animals: C57BL/6 mice used in this study were maintained on a 12-h light/dark cycle (lights on from 7:00 to 19:00) at room temperature of 22 ± 2°C. The mice were kept with free access to food and water. All the mice used in this study were age-matched littermates.

Anesthetic and hydrogen treatment: The mice at postnatal day 6 (P6) during the brain developmental stage were taken out from the maternal cage and immediately thereafter placed in a humid chamber that has manipulating gloves. Air, oxygen (besides the oxygen contained in the "air"), hydrogen and sevoflurane were mixed to prepare an anesthetic mixed gas containing 30% oxygen, 1. 3% hydrogen and 3% sevoflurane as final concentrations, and the anesthetic mixed gas was administered via inhalation to the mice. The total gas flow was 2 L/min and the administration time of the anesthetic was 6 hours. The fractions of oxygen and the anesthetic were measured by a gas analysis system (Capnomac Ultima, GE Healthcare, Tokyo, Japan). The hydrogen gas concentration was measured by gas chromatography in a company called Breath Lab CO. (Nara, Japan). During the exposure to the anesthetic, the mice were kept warm on a mat heated at 38 ± 1°C.

### <Example 2>

The same procedures as described in Example 1 were performed except that air, oxygen (besides the oxygen contained in the "air"), hydrogen and sevoflurane were mixed to prepare an anesthetic mixed gas containing 30% oxygen, 0.6% hydrogen and 3% sevoflurane as final concentrations.

### <Example 3>

The same procedures as described in Example 1 were performed except that air, oxygen (besides the oxygen contained in the "air"), hydrogen and sevoflurane were mixed to prepare an anesthetic mixed gas containing 30% oxygen, 0.3% hydrogen and 3% sevoflurane as final concentrations.

### <Example 4>

The same procedures as described in Example 1 were performed except that air, oxygen (besides the oxygen contained in the "air"), hydrogen and desflurane were mixed to prepare an anesthetic mixed gas containing 30% oxygen, 1.3% hydrogen and 5.7% desflurane as final concentrations.

### <Example 5>

The same procedures as described in Example 1 were performed except that air, oxygen (besides the oxygen contained in the "air") and hydrogen were mixed to prepare a mixed gas containing 30% oxygen and 1.3% hydrogen, and inhalational administration of the mixed gas was performed simultaneously with intraperitoneal administration of propofol (100 mg/kg i.p.).

### <Example 6>

The same procedures as described in Example 1 were performed except that air, oxygen (besides the oxygen contained in the "air"), hydrogen and sevoflurane were mixed to prepare an anesthetic mixed gas containing 30% oxygen, 1.3% hydrogen and 2% sevoflurane as final concentrations.

### <Comparative Example 1>

The same procedures as described in Example 1 were performed except that air, oxygen (besides the oxygen contained in the "air") and sevoflurane were mixed to prepare an anesthetic mixed gas containing 30% oxygen and 3% sevoflurane as final concentrations.

### <Comparative Example 2>

The same procedures as described in Example 1 were performed except that air, oxygen (besides the oxygen contained in the "air") and desflurane were mixed to prepare an anesthetic mixed gas containing 30% oxygen and 5.7% desflurane as final concentrations.

### <Comparative Example 3>

The same procedures as described in Example 1 were performed except that air and oxygen (besides the oxygen contained in the "air") were mixed to prepare a mixed gas containing 30% oxygen, and inhalational administration of the mixed gas was performed simultaneously with intraperitoneal administration of propofol (100 mg/kg i.p.).

### Test Example 1-A

Purification of protein extracts: Preparation of protein extracts was performed as described in Kodama M. et al., Anesthesiology, 2011; 115: 979-991, followed by western blotting. The procedures are described briefly in the following. The forebrain of each mouse was quickly removed and homogenized in a 4-fold excess of a homogenization buffer containing 50 mM Tris HCl (pH 7.4), 150 mM NaCl, 1% NP-40, 0.5% sodium deoxycholate, a protease inhibitor cocktail (Complete; Roche Diagnostics, Penzberg, Germany) and phosphatase inhibitors (20 mM glycerophosphate, 1 mM Na₃VO₄ and 2 mM NaF). Then, the homogenate was centrifuged at 15,000 g at 4°C for 30 minutes. The supernatant was separated and stored at -80°C until use. The protein concentration of each sample was measured with the use of a bicinchoninic acid protein assay kit (Pierce, Rockford, IL).

Western blot analysis: Western blotting was performed according to the method described in Kodama M. et al., Anesthesiology, 2011; 115: 979-991. The procedures are described briefly in the following. The homogenates were subjected to sodium dodecyl sulfate polyacrylamide gel electrophoresis. Then, the proteins were transferred onto a polyvinylidene fluoride membrane (Immobilon-P; Millipore, Bedford, MA). The blots were immunoreacted with an anti-poly(adenosine diphosphate ribose) polymerase (anti-PARP) antibody (rabbit polyclonal; Cell Signaling Technology) and an anti-β-actin antibody (mouse monoclonal; Sigma, St. Louis, MO). The blots were then incubated with a peroxidase-conjugated secondary antibody. The protein bands were visualized by a chemiluminescence detector (SuperSignal West Pico; Pierce). The band intensities of the cleaved PARP were quantified and normalized to β-actin. Comparison of the groups was performed using a two-way ANOVA followed by the Bonferroni post-hoc test (n = 3 to 6 mice per group).

The extracts from the forebrain were analyzed by Western blotting using an antibody against cleaved PARP (biomarker of apoptotic cell death). The analysis results are shown in Fig. 1A. The quantified band intensities of the cleaved PARP are shown in Fig. 1B. As shown in Figs. 1A and 1B, the immunoreactivity for the cleaved PARP in the brain of the mice exposed only to a gas containing 30% oxygen or to a gas containing 30% oxygen and 1.3% hydrogen was below the detection level, but the reaction producing cleaved PARP was induced in the mice exposed to a gas containing 30% oxygen and 3% sevoflurane for 6 hours (Comparative Example 1) . Meanwhile, in the mice exposed to a gas containing 30% oxygen, 1.3% hydrogen and 3% sevoflurane (Example 1), the immunoreactivity for cleaved PARP was remarkably reduced as compared with the mice exposed to sevoflurane in a gas containing 30% oxygen (that is, hydrogen gas inhibited cleavage of PARP), and thus 1.3% hydrogen gas was shown to inhibit sevoflurane exposure-induced neuronal apoptosis in neonatal mice. Significant differences between these groups were found by a two-way ANOVA, and the primary effect of hydrogen inhalation (F = 12.17, P < 0.01), the primary effect of sevoflurane administration (F = 45.66, P < 0.0001), and interaction (hydrogen administration x sevoflurane administration; F = 15.28, P < 0.01) were found.

When the quantity of the cleaved PARP in Comparative Example 1 was set to 100%, the relative quantities of the cleaved PARP in Examples 1, 2 and 3 were lower by about 45%, by about 50% and by about 55%, respectively, and significant decreases were observed in the quantity of the cleaved PARP. In Example 6, neuronal apoptosis was significantly reduced as with Example 1. These results showed that the present invention can inhibit sevoflurane exposure-induced neuronal apoptosis by as high as 40% or more as compared with the case where hydrogen is not used.

### Test Example 1-B

The evaluation of Example 4 and Comparative Example 2 was performed in the same manner as in Test Example 1-A. When the quantity of the cleaved PARP in Comparative Example 2 was set to 100%, the relative quantity of the cleaved PARP in Example 4 was lower by 47.7% and a significant decrease was observed in the quantity of the cleaved PARP. This result showed that the present invention inhibits desflurane exposure-induced neuronal apoptosis by as high as 45% or more.

### Test Example 1-C

The evaluation of Example 4 and Comparative Example 3 was performed in the same manner as in Test Example 1-A. When the quantity of the cleaved PARP in Comparative Example 3 was set to 100%, the relative quantity of the cleaved PARP in Example 5 was lower by 55.1% and a significant decrease was observed in the quantity of the cleaved PARP. This result showed that the present invention inhibits propofol exposure-induced neuronal apoptosis by as high as 50% or more.

### Test Example 2

Histopathological studies: Immunohistochemical staining was performed according to the method described in Kodama M. et al. , Anesthesiology, 2011; 115: 979-991 and Satoh Y. et al. , J Neurosci, 2011; 31: 11953-11967. The procedures are described briefly in the following. Mice were transcardially perfused with a 0.1 M phosphate buffer containing 4% paraformaldehyde. In each mouse, the skull was opened and the head portion was immersed in the same buffer as above for at least 2 hours. Then, the brain was removed from the skull, and paraffin-embedded sections (5-µm thick) of the brain were prepared and histopathologically analyzed. The sections were deparaffinized in xylene and hydrated using a graded ethanol series according to the established method. For antigen retrieval, the deparaffinized sections were immersed in an antigen retrieval solution (Antigen Unmasking Solution; Vector Laboratories, Burlingame, CA), and heated in an autoclave (121°C) for 5 minutes. Then, the sections were treated with a blocking reagent (Protein Block, Serum-Free; Dako, Glostrup, Denmark) for 30 minutes to reduce background staining. Then, the sections were incubated with a primary antibody in a humid chamber at 4°C overnight. The primary antibodies used in this study were an anti-active caspase-3 antibody (rabbit polyclonal; Cell Signaling Technology, Beverly, MA) and an anti-4-hydroxy-2-nonenal (anti-4-HNE) antibody (mouse monoclonal; Japan Institute for the Control of Aging, Shizuoka, Japan).

For bright field staining, the sections were then incubated with a peroxidase-conjugated secondary antibody (Dako EnVision+ system; Dako). Immunoreactivity was revealed using 3,3-diaminobenzine tetrachloride (DAB, Vector Laboratories) according to the manufacturer's protocol. Finally, the sections were counterstained with hematoxylin. For fluorescent staining, the sections were incubated with an Alexa Fluor 546-conjugated anti-mouse IgG antibody (Life Technologies, Eugene, OR).

As described in Kodama M. et al. , Anesthesiology, 2011; 115: 979-991, terminal deoxynucleotidyl transferase-mediated nick-end labeling (TUNEL) assay was performed using an in situ apoptosis detection kit (ApopTag; Chemicon, Temecula, CA) according to the manufacturer's protocol. DAB was used to reveal reactivity. The sections were counterstained with hematoxylin.

Each test was performed using samples obtained from each group consisting of 8 to 10 mice exposed to anesthesia under the same conditions as in Example 1 or Comparative Example 1. An examiner blinded to the treatment conditions counted the number of active caspase-3-positive or TUNEL-positive cells.

Histological analysis was performed using an antibody against active caspase-3 (another biomarker of apoptotic cell death) (Fig. 2). In order to examine the activity of caspase-3, the sections were subjected to immunochemical staining. Since the western blot analysis showed that the apoptosis level in the mice exposed to a gas containing 30% oxygen and 1.3% hydrogen was the same as that in the mice exposed to a gas containing 30% oxygen as described above, histological quantification was performed only on the following three groups:
(i) a gas containing 30% oxygen (hereinafter referred to as control),
(ii) a gas containing 30% oxygen and 3% sevoflurane (hereinafter referred to as sevoflurane) (Comparative Example 1), and
(iii) a gas containing 30% oxygen, 1.3% hydrogen and 3% sevoflurane (hereinafter referred to as sevoflurane + hydrogen)

### (Example 1).

The 6-hour sevoflurane exposure (Comparative Example 1) induced a remarkable increase in the number of active caspase-3-positive cells in some regions in the brain immediately after the end of the 6-hour anesthesia as compared with the sham control (Fig. 2B). Meanwhile, in the mice exposed to sevoflurane + hydrogen (Example 1), the number of active caspase-3-positive cells was remarkably reduced as compared with the exposure to sevoflurane alone (Figs. 2 and 3). Figs. 2 and 3 clearly showed that hydrogen gas alleviates sevoflurane-induced neuronal apoptosis in developing brains. In order to measure apoptotic cell death at the cellular level, we also performed the TUNEL assay (Fig. 4). The pattern of TUNEL staining after the 6-hour anesthesia was similar to that of active caspase-3 staining. These results showed that 1.3% hydrogen remarkably reduces sevoflurane exposure-induced neuronal apoptosis in neonates.

Hydroxy radicals react with lipids to generate lipid peroxides including 4-HNE. For this reason, 4-HNE is widely used as a marker of lipid peroxidation and oxidative stress. Fig. 5 shows that the 6-hour sevoflurane exposure (Fig. 5B, Comparative Example 1) induced more lipid peroxidation in neurons as compared with the sham control (Fig. 5A). Meanwhile, 4-HNE staining in the brains of the mice exposed to sevoflurane + hydrogen (Example 1) (Fig. 5C) was remarkably reduced as compared with the exposure to sevoflurane alone (Fig. 5B). These results showed that hydrogen reduces brain oxidative stress induced by 3% sevoflurane exposure in neonatal mice.

### Test Example 3

Behavioral tests: All mice used for behavioral studies were age-matched male littermates exposed to anesthesia under the same conditions as in Example 1 or Comparative Example 1. At 3 weeks of age, these mice were weaned and housed in groups of three or four animals per cage. At predetermined ages, they were subjected to behavioral tests to evaluate anesthetic effects. The behavioral tests included an open field test as a control for the evaluation of long-term memory impairment, a Y-maze spontaneous alternation test for the evaluation of short-term memory impairment, fear conditioning tests for the evaluation of long-term memory impairment, and sociability tests. As for the sociability tests, in addition to a social interaction test, a novelty test and an olfactory test were performed as controls. The movement of each mouse was monitored and analyzed using a computer-operated video tracking system (SMART; Barcelona, Spain). In the tests, an apparatus with arms was used and the number of entry of all four legs of the animal into the arm was counted. The apparatus was cleaned for every trial. All apparatuses used in this study were manufactured by O'Hara & Co. LTD. (Tokyo, Japan). The same set of mice was subjected to all the tests.

Open field test: Emotional responses to a novel environment were measured in an open field test according to the method described in Satoh Y. et al. , J Neurosci, 2011; 31: 11953-11967. Activity was measured as the total travel distance (meter) in 10 minutes. The test was performed on 12-week-old mice. The results are shown in Fig. 6A.

Y-maze spontaneous alternation test: For evaluation of spatial working memory, a Y-maze test was performed according to the method described in Satoh Y. et al., J Neurosci, 2011; 31: 11953-11967. The test used a symmetrical acrylic Y maze consisting of three arms (25 x 5 cm) spaced 120 degrees apart with a transparent wall of 15 cm in height. Each mouse was placed on the center of the Y maze, and allowed to freely explore the maze for 8 minutes. The total number of arm entries and the number of triads were recorded. The percentage of alternation was obtained by dividing the number of triads (three consecutive entries into the three different arms) by the maximum possible number of alternations (the total number of arm entries minus 2), followed by multiplying the resulting value by 100. The test was performed on 12-week-old mice. The results are shown in Fig. 6B.

Fear conditioning test: A fear conditioning test was performed according to the method described in Satomoto M. et al., Anesthesiology, 2009; 110: 628-637. The procedures are described briefly in the following. Each mouse was placed in a special cage and presented with 80 dB white noise of 20-second duration. At the 20th second of the stimulus presentation, a 1-sec, 1-mA footshock was given, and this stimulus pairing was repeated 3 times at intervals of 1 minute. At 24 hours after the repetitive stimulation, the mouse was returned to the cage, and the total time of freezing responses (a state of the absence of movement in any parts of the body for one second) was measured for 5 minutes (contextual fear conditioning test). At 48 hours after the repetitive stimulation, the mouse was placed into a cage of a different shape in a completely different place and presented with white noise only, and the total time of freezing responses was measured for 3 minutes (auditory (cued) fear conditioning test). The freezing response was recorded in the video tracking system and regarded as a measure of fear memory. The test was performed on 13-week-old mice. The mice subjected to this test were not used for any further testing (the same set of mice that had been used in the open field test and the Y-maze spontaneous alternation test was subjected to this test). Fig. 6C shows the measurement results of freezing responses observed in the mice placed in the conditioning chamber 24 hours after the conditioning (contextual fear response). Fig. 6D shows the measurement results of freezing responses observed in the mice placed in a cage of a different shape in a completely different place under white noise presentation 48 hours after the conditioning.

Sociability tests: The tests performed to assess sociability were the following three tests: a social interaction test, a novelty test and an olfactory test.

In order to examine social interaction capability, a sociability test was performed according to the method described in Satoh Y. etal., J Neurosci, 2011; 31: 11953-11967. The preference for interaction with an animate target (caged adult mouse) versus an inanimate target (caged dummy mouse) was examined in an open field chamber. The animate or inanimate target was placed in a cylindrical cage so that olfactory interaction and minimal tactile interaction were allowed. The cylindrical cage has a height of 10 cm, a diameter of 9 cm and bars spaced 7 mm apart. Sniffing directed at the cage was monitored under 70 lux lighting conditions for 10 minutes and then scored. The test was performed on 12-week-old mice (control: n = 18; sevoflurane: n = 20; sevoflurane + hydrogen: n = 19). All the animate targets used were wild type male mice. The results are shown in Fig. 7A.

Olfactory test: An olfactory test was performed as described in Satoh Y. et al. , J Neurosci, 2011; 31: 11953-11967, with some modifications. The procedures are described briefly in the following. Mice were habituated to the flavor of a novel food (blueberry cheese) on the first day. After 48-hour food deprivation, a piece of blueberry cheese was buried under 2 cm of bedding in a clean cage, and the time required to find the buried food was measured. The test was performed on 12-week-old mice (the same set of mice that had been used in the above sociability test was subjected to this test). The results are shown in Fig. 7B.

Novelty test: A novelty test was performed according to the method described in Satoh Y. et al., J Neurosci, 2011; 31: 11953-11967. Mice were individually housed and the total time spent interacting with an inanimate novel object (a small red tube) in 10 minutes was measured. The test was performed on 12-week-old mice (the same set of mice that had been used in the sociability test and the olfactory test was subjected to this test). The results are shown in Fig. 7C.

In the open field test performed for the evaluation of emotional responses to a novel environment, no statistically significant differences in the total travel distance over 10 minutes were observed between the groups (control: n = 18; sevoflurane: n = 20; sevoflurane + hydrogen: n = 19) (Fig. 6A). Therefore, it was shown that general anesthetics do not affect emotional responses.

Working memory is the ability to temporarily hold information, which is essential for carrying out complex cognitive tasks (Saxe MD et al., Proc Natl Acad Sci USA 2006; 103: 17501-17506, and Jones MW, Curr Mol Med 2002; 2: 639-647). In the Y-maze test performed for the evaluation of spatial working memory (Fig. 6B), no statistically significant differences were observed between the groups (the same set of mice that had been used in the open field test was subjected to this test). Therefore, it was shown that general anesthetics do not affect short-term memory.

In order to evaluate the effect of hydrogen on long-term memory impairment caused by neonatal exposure to sevoflurane, mice neonatally exposed to sevoflurane together with hydrogen (Example 1) or without hydrogen (Comparative Example 1) were subjected to the fear conditioning test in adulthood (Figs. 6C and 6D). In the contextual fear conditioning test (Fig. 6C), freezing responses in the contextual test session 24 hours after the repetitive stimulation were remarkably reduced in the sevoflurane-exposed mice (Comparative Example 1) as compared with the control animals (one-way ANOVA, F = 7.22, P = 0.0017; Newman-Keuls post-hoc test, P < 0.01 for control vs. sevoflurane), and neonatal exposure to sevoflurane was shown to cause long-term memory impairment in adulthood. In contrast, the mice exposed to sevoflurane + hydrogen (Example 1) showed improved behaviors as compared with the mice exposed to sevoflurane only (Comparative Example 1) (Newman-Keuls post-hoc test, P < 0.01 for sevoflurane vs. sevoflurane + hydrogen) and almost the same performance as the control did (Newman-Keuls post-hoc test, P < 0.05 for control vs. sevoflurane + hydrogen). In the auditory (cued) fear conditioning test (Fig. 6D), freezing responses in the auditory test session 48 hours after conditioning were remarkably reduced in the sevoflurane-exposed mice (Comparative Example 1) as compared with the control (one-way ANOVA, F = 12.08, P = 0.0001; Newman-Keuls post-hoc test, P < 0.001 for control vs. sevoflurane). In contrast, the mice exposed to sevoflurane + hydrogen (Example 1) showed better behaviors as compared with the mice exposed to sevoflurane only (Comparative Example 1) (Newman-Keuls post-hoc test, P < 0.001 for sevoflurane vs. sevoflurane + hydrogen) and almost the same performance as the control did (Newman-Keuls post-hoc test, P < 0.05 for control vs. sevoflurane + hydrogen).

These results showed that the kind of memory impairment caused by neonatal exposure to general anesthetics is long-term memory impairment and that hydrogen prevents and/or alleviates such memory impairment.

Mice are a social species and exhibit behavioral social interaction (Kamsler A et al. , Mol Neurobiol 2004; 29: 167-178). We previously reported that mice neonatally exposed to sevoflurane showed social behavioral deficits in adulthood (Satomoto M. et al., Anesthesiology 2009; 110: 628-637). This time, in order to examine whether hydrogen gas can inhibit the social behavioral deficits caused by neonatal exposure to sevoflurane, the sociability tests were performed on mice (Fig. 7).

In the interaction test using an animate or inanimate target, all the groups spent much more time interacting with the animate target than with the inanimate target (t-test, P < 0.001 for every comparison). However, the mice neonatally exposed to sevoflurane (Comparative Example 1) spent less time interacting with the animate target than the control did. The mice subjected to simultaneous administration of sevoflurane with hydrogen (Example 1) showed almost the same behaviors as the control did, and the simultaneous administration of sevoflurane with hydrogen was shown to prevent the social behavioral deficits caused by sevoflurane (Fig. 7A). These results were confirmed by a one-way ANOVA (F = 6. 12, P = 0.004; Newman-Keuls post-hoc test, P < 0.01 for control vs. sevoflurane, P < 0.05 for control vs. sevoflurane + hydrogen). It is unreasonable to say that the differences in social interaction described above were attributed to impaired olfactory sensation or loss of interest in novelty because no remarkable differences were observed between the sevoflurane administration group (Comparative Example 1) and the sevoflurane + hydrogen administration group (Example 1) in the olfactory test (one-way ANOVA, F=0.50, P=0.71, Fig. 7B) and the novelty test (one-way ANOVA, F = 0.04, P = 0.96, Fig. 7C). Therefore, it can be said that hydrogen can inhibit social behavioral deficits caused by neonatal exposure to sevoflurane.

### INDUSTRIAL APPLICABILITY

The present invention, which uses a general anesthetic in combination with hydrogen, makes it possible to provide a medicine capable of preventing and/or alleviating an anesthetic-induced neurological deficit in the brain (for example, in the developing brain). Further, the medicine is convenient, free from side effects, efficacious and inexpensive, and therefore the present invention can provide a medicine for general anesthesia which is effective in obstetric and pediatric care.

## Claims

1. A medicine for a human or a non-human animal, comprising a combination of a general anesthetic and hydrogen.

2. A medicine for general anesthesia of a human or a non-human animal, **characterized in that** a general anesthetic and hydrogen are administered in combination.

3. The medicine according to claim 1 or 2, wherein the medicine is used for prevention and/or alleviation of an anesthetic-induced neurological deficit.

4. The medicine according to claim 3, wherein the anesthetic-induced neurological deficit is associated with neuronal apoptosis.

5. A medicine for prevention and/or alleviation of an anesthetic-induced neurological deficit, comprising a general anesthetic, the general anesthetic being used in combination with hydrogen.

6. The medicine according to any one of claims 1 to 5, wherein the general anesthetic is an inhalational anesthetic or a liquid intravenous anesthetic and the hydrogen is hydrogen gas.

7. The medicine according to claim 6, wherein the concentration of the hydrogen gas in the medicine is 0.15 to 7% (v/v).

8. The medicine according to any one of claims 1 to 7, wherein the medicine is for a fetus, a neonate, an infant, a preschool child, a child or an elderly adult.

9. The medicine according to any one of claims 1 to 8, wherein the general anesthetic is one or more kinds of anesthetics selected from the group consisting of nitrous oxide, isoflurane, enflurane, methoxyflurane, sevoflurane, desflurane, diethyl ether, propofol and midazolam.

10. The medicine according to any one of claims 3 and 5 to 9, wherein the anesthetic-induced neurological deficit is a neuromotor deficit, a neurocognitive deficit, a psychocognitive deficit or autism.

11. A method for preparing a medicine for prevention and/or alleviation of an anesthetic-induced neurological deficit, the method using a general anesthetic in combination with hydrogen.

12. The method according to claim 11, wherein the anesthetic-induced neurological deficit is associated with neuronal apoptosis.

13. The method according to claim 11 or 12, wherein the general anesthetic is an inhalational anesthetic or a liquid intravenous anesthetic and the hydrogen is hydrogen gas.

14. The method according to claim 13, wherein the concentration of the hydrogen gas in the medicine is 0.15 to 7% (v/v).

15. The method according to any one of claims 11 to 14, wherein the medicine is for a fetus, a neonate, an infant, a preschool child, a child or an elderly adult.

16. Use of a general anesthetic for production of a medicine for general anesthesia used in combination with hydrogen.

17. Use of a general anesthetic and hydrogen for production of a medicine comprising a combination of a general anesthetic and hydrogen.

18. Use of a general anesthetic and hydrogen for production of a medicine for prevention and/or alleviation of an anesthetic-induced neurological deficit.

19. The use according to claim 18, wherein the anesthetic-induced neurological deficit is associated with neuronal apoptosis.

20. The use according to any one of claims 16 to 18, wherein the general anesthetic is an inhalational anesthetic or a liquid intravenous anesthetic and the hydrogen is hydrogen gas.

21. The use according to claim 20, wherein the concentration of the hydrogen gas in the medicine is 0.15 to 7% (v/v).

22. The use according to any one of claims 16 to 18, wherein the use is for a fetus, a neonate, an infant, a preschool child, a child or an elderly adult.

23. The use according to any one of claims 16 to 18, wherein the general anesthetic is one or more kinds of anesthetics selected from the group consisting of nitrous oxide, isoflurane, enflurane, methoxyflurane, sevoflurane, desflurane, diethyl ether, propofol and midazolam.

24. The use according to claim 18, wherein the anesthetic-induced neurological deficit is a neuromotor deficit, a neurocognitive deficit, a psychocognitive deficit or autism.

25. A method for preventing and/or alleviating an anesthetic-induced neurological deficit, comprising the step of administering a general anesthetic in combination with hydrogen to a subject.

26. The method according to claim 25, wherein the general anesthetic is an inhalational anesthetic or a liquid intravenous anesthetic and the hydrogen is hydrogen gas.

27. The method according to claim 26, wherein the concentration of the hydrogen gas in a medicine is 0.15 to 7% (v/v).

28. The method according to claim 25, wherein the subject is a fetus, a neonate, an infant, a preschool child, a child or an elderly adult.

29. The method according to claim 25, wherein the general anesthetic is one or more kinds of anesthetics selected from the group consisting of nitrous oxide, isoflurane, enflurane, methoxyflurane, sevoflurane, desflurane, diethyl ether, propofol and midazolam.

30. The method according to claim 25, wherein the anesthetic-induced neurological deficit is a neuromotor deficit, a neurocognitive deficit, a psychocognitive deficit or autism.

31. The method according to claim 25, wherein the anesthetic-induced neurological deficit is associated with neuronal apoptosis.
